Europäisches Patentamt

European Patent Office

Office européen des brevets

(11) Publication number: **0 067 026**
A1

# EUROPEAN PATENT APPLICATION

(21) Application number: 82302804.8

(22) Date of filing: 01.06.82

(51) Int. Cl.³: **C 12 N 15/00,** C 12 P 21/02,
C 12 N 1/20, C 12 R 1/19

(30) Priority: 01.06.81 US 269187

(43) Date of publication of application: 15.12.82
Bulletin 82/50

(84) Designated Contracting States: **BE CH DE FR GB IT LI
NL SE**

(71) Applicant: **THE BOARD OF TRUSTEES OF THE
MICHIGAN STATE UNIVERSITY, 238 Administration
Building Michigan State University, East Lansing
Michigan 48824 (US)**

(72) Inventor: **Rottman, Fritz Max, 28500 Gates Mills
Boulevard, Pepper Pike Ohio (US)**
Inventor: **Nilson, John Helmer, 2324 Canterbury Road,
University Heights Ohio (US)**

(74) Representative: **Perry, Robert Edward et al, GILL
JENNINGS & EVERY 53-64 Chancery Lane, London
WC2A 1HN (GB)**

(54) Process for cloning bovine hormone gene, and plasmids and plasmid hosts for use therein.

㉝ The DNA sequence coding for bovine growth hormone, which in the bovine genome is present as only one out of approximately one million genes, has been isolated, and inserted into a small DNA plasmid which can be easily replicated in E. coli. Thus it is possible to obtain milligram amounts of a purified DNA sequence coding for bovine growth hormone. This purified DNA can be used either in its present form, or by insertion into another plasmid, for expression in micro-organisms to produce large amounts of bovine growth hormone. This hormone is useful to stimulate the growth and milk production of cattle.

EP 0 067 026 A1

PROCESS FOR CLONING BOVINE GROWTH HORMONE GENE,
AND PLASMIDS AND PLASMID HOSTS FOR USE THEREIN

This invention relates to a process for cloning bovine growth hormone gene, and to plasmids and plasmid hosts for use in the process.

Bovine growth hormone (BGH) is a 191 amino-acid polypeptide, synthesised initially as a pregrowth hormone containing a signal peptide of 26 amino-acids; see Hunt et al, "Atlas of Protein Sequence and Structure", ed. M.O. Dayhoff (National Biomedical Research Foundation, Washington, D.C.) Vol. 5, Suppl. 2, pp. 113-139, and Lingappa et al, Proc. Natl. Acad. Sci. 74 (1977) 2432-2436. The 3'-untranslated region of BGH has been shown to be 104 nucleotides in length; see Sasavage et al, Biochemistry 19 (1980) 1737-1743. It has been shown that the RNA of bovine prolactin (PRL) and BGH comprise most of the mRNA population of the bovine anterior pituitary; see Nilson et al, J. Biol. Chem. 254 (1979) 1516-1520.

Miller et al, J. Biol. Chem. 255 (1980) 7521-7524, and Keshet et al, Nucleic Acids Res. 9 (1981), have reported the cloning of cDNA. The strategy taken by Keshet et al. in screening for BGH clones was indirect and required a number of manipulations to identify growth hormone clones. The approach taken by Miller et al in screening for clones was less complicated, but still involved a couple of steps.

The BGH sequence cloned by Keshet et al contains only part of the signal peptide, and does not contain any of the 5'-untranslated region. In the BGH clone isolated by Miller et al, at the position coding for amino-acid 34 of the growth hormone (Arg), the sequence is CGU.

According to the present invention, a process for preparing BGH comprises culturing a bacterium modified to contain a nucleotide sequence coding for the amino-acid sequence of BGH. The present invention also provides microorganisms so modified, and DNA transfer vectors comprising such nucleotide sequences.

The invention will now be described with reference to specific embodiments, which have been shown to provide BGH differing, at least at the position coding for amino-acid 34, from that prepared by Miller et al.

BRIEF SUMMARY OF THE INVENTION

Poly(A)-containing RNA from the bovine anterior pituitary is used as a template for the enzymatic synthesis of double-stranded cDNA. The resulting double-stranded cDNA is inserted into the Pst I site of pBR322 with the oligo(dG)-oligo(dC) tailing technique and subsequently cloned in E. coli $_\chi$1776. Clones containing sequences complementary to growth hormone (GH) mRNA are identified by colony hybridization with partially purified growth hormone cDNA. An approximately 841 base pair sequence from a growth hormone positive clone is shown to contain the coding information for authentic bovine growth hormone. The cloned GH cDNA includes the sequence for most of the 5' untranslated region, all of the coding regions for the signal peptide and the growth hormone, and all of the 3' untranslated region. The inserted growth hormone cDNA is in the same orientation as that of the β-lactamase gene of plasmid pBR322.

The symbols and abbreviations used herein are as follows:

DNA - deoxyribonucleic acid

RNA - ribonucleic aid

cDNA - complementary DNA (enzymatically synthesized from an mRNA sequence)

mRNA - messenger RNA

tRNA - transfer RNA

dATP - deoxyadenosine triphosphate

dGTP - deoxyguanosine triphosphate

dCTP - deoxycytidine triphosphate

dTTP - deoxythymidine triphosphate

A - Adenine

T - Thymine

G - Guanine

C - Cytosine

Tris - 2-Amino-2-hydroxyethyl-1,3-propanediol

EDTA - ethylenediamine tetraacetic acid

ATP - adenosine triphosphate

TTP - thymidine triphosphate

DETAILED DESCRIPTION OF THE INVENTION

Bovine pituitary growth hormone cDNA is cloned into the Pst I site of plasmid pBR322 by the oligo(dG)-oligo(dC) tailing technique.

The GH plasmid pLG23, which is approximately 841 bp in length, is first identified by colony hybridization with GH enriched cDNA, and then further characterized by southern transfer, and by selective hybridization to GH mRNA. The GH plasmid is characterized by restriction enzyme analysis and by partial sequencing. Our analysis shows that the growth hormone plasmid pLG23 has the following structure: The approximately 841 bp inserted sequence consists of three Pst I fragments. One fragment of 57 bp at the 5' end of the insert (in regard to the coding strand), a 338 bp fragment in the center of the sequence, and a fragment of approximately 446 bp at the 3' end of the insert. Plasmid pLG23 should contain a 5' dC-dG tail assumed to be about 15 bp long; a portion of the 5' uncoding region, assumed to be approximately 30 bp in length, a complete signal peptide coding region, expected to be 78 bp long, a complete growth hormone coding region, expected to be 573 bp long, a complete 3' uncoding region expected to be 104 bp long, a portion of the 3' poly(A) tail expected to be approximately 26 bp, and a 3' dC-dG tail assumed to be approximately 15 bp long. The growth hormone sequence is inserted within the β lactamase gene of pBR322 in a 5' to 3' counterclockwise orientation which is the direction of transcription and translation of the β lactamase, thus giving rise probably to a fusion protein which, if read in frame, will have also some GH characteristics. The growth hormone sequence that we have cloned is either full length or very close to full length GH mRNA. We believe that it is possible to get such a long insert by using mild S1 treatment on the double-stranded cDNA and size selection of the insert sequences before transformation.

Materials. All radioactive nucleoside triphosphates can be obtained from Amersham and New England Nuclear; terminal transferase from Enzo Biochem. Inc.; proteinase K obtained from E.M. Labs; T$_4$-polynucleotide kinase from New England Biolabs; S$_1$-nuclease from Miles; bacterial alkaline phosphatase from Worthington; and lysozyme from Sigma. Reverse transcriptase is available from various sources, e.g. Bethesda Research Laboratories. All restriction enzymes can be purchased from Bethesda Research Laboratories and New England Biolabs. The digestions with restriction enzymes can be carried out using the conditions suggested by the supplier for each enzyme.

Following are examples which illustrate procedures, including the best mode, for practicing the invention. These examples should not be construed as limiting. All percentages are by weight and all solvent mixture proportions are by volume unless otherwise noted.

Example 1        Isolation of Bovine Pituitary mRNA

Polysomes are prepared from fresh bovine anterior pituitaries by a modification of the magnesium precipitation method as described by Palmiter [Palmiter, R.D. (1974) Biochemistry 13, 3606-3615]. Modifications included the use of 0.2% instead of 2.0% Triton X-100, and a brief proteinase K treatment (250 µg/ml, 37°C, 30 minutes in buffer containing 1.0% SDS) of the isolated polysomes prior to phenol-chloroform extraction. Poly(A)-containing RNA is isolated from total polysomal RNA by oligo(dT)-cellulose chromatography as described previously [Desrosiers, R.C., Friderici, K.H., and Rottman, F.M. (1975) Biochemistry 14, 4367-4376].

Growth hormone and prolactin mRNA are purified from the poly(A)-containing RNA by centrifugation on 5-20% (w/w) sucrose gradients in 10 mM Tris (pH 7.5), 1 mM EDTA, and 0.2% SDS. Centrifugation is performed at 35,000 rpm for 12 hours at 22°C in a Beckman SW-41 rotor. The gradients are fractionated and the absorbance at 260 nm determined with a Gilford 240 recording spectrophotometer. The RNA from each fraction is recovered by ethanol precipitation and translated in the wheat germ cell-free system as previously described [Nilson, J.H., et al, supra]. Fractions containing GH or PRL mRNA's are each pooled and centrifuged a second time as described above for further purification.

Example 2        Synthesis of Single-Stranded cDNA

The synthesis of cDNA to poly(A)-containing RNA is carried out essentially as described by Myers et al. [Myers, J.C., Spiegelman, S., and Kacian, D.L. (1977) Proc. Natl. Acad. Sci. USA 74, 2840-2843]. The components of the reaction are: 50 mM Tris (pH 8.3), 37.5 mM KCl, 8 mM MgCl$_2$, 250 µM each of dATP, dGTP, dTTP, [$^3$H]-dCTP (0.08 Ci/mMole), 400 µM dithiothreitol, 4 mM sodium pyrophosphate, 5 µg/ml oligo(dT)$_{12-18}$, 100 µg/ml poly(A)-containing RNA, and 300 units/ml reverse transcriptase. The 500 µl reaction is incubated at 37°C for one hour, extracted with chloroform-isoamyl alcohol (24:1), and then chromatographed over Sephadex G-50 in 20 mM Tris (pH 7.5), 2 mM EDTA, 100 mM NaCl, and 0.2% SDS. The fractions containing cDNA are pooled and ethanol precipitated. The RNA is hydrolyzed from the cDNA by incubation in 0.6 N NaOH, 0.01 M EDTA for two hours at 37°C. The mixture is neutralized with 6 N HCl and the cDNA precipitated by

adding MgCl$_2$ to 0.01 M, 18S rRNA to 10 µg/ml, and two volumes of ethanol (-80°C, two hours). The final cDNA pellet is dissolved in water and stored at -20°C.

When cDNA is synthesized from GH or PRL enriched mRNA for use in colony hybridization, the RNA concentration is lowered to 20 µg/ml, and 10 µM [$\alpha$-$^{32}$p]-dCTP (200 Ci/mMole) is used in place of [$^3$H]-dCTP.

Example 3    Synthesis of Double-Stranded cDNA

The reaction conditions for synthesis of the second cDNA strand are: 50 mM Tris (pH 8.3), 37.5 mM KCl, 8 mM MgCl$_2$, 250 µM each of dATP, dGTP, and dTTP, 100 µM [$^3$H]-dCTP (2.5 Ci/mMole), 10 µg/ml cDNA, and 400 units/ml of reverse transcriptase. The reaction is incubated at 37°C for two hours, extracted with chloroform-isoamyl alcohol and chromatographed on Sephadex G-50, as described above. The fractions containing the cDNA are pooled, adjusted to 40 µg/ml with yeast tRNA and ethanol precipitated. The 3'-terminal hairpin loop is opened by digesting the cDNA with SI-nuclease at room temperature for 30 minutes. The components of this reaction are 0.3 M NaCl, 0.05 M potassium acetate (pH 4.5), 1 mM ZnSO$_4$, 5 µg/ml cDNA, and 48 units S$_1$-nuclease per µg of total nucleic acid. In order to terminate the S$_1$ digestion, Tris (pH 7.4), EDTA, and tRNA are added to 80 mM, 20 mM, and 50 µg/ml, respectively. The reaction mixture is then extracted with buffer saturated phenol-chloroform, and the cDNA is ethanol precipitated.

Example 4    Size Selection of the S$_1$ Treated Double-Stranded cDNA

The double-stranded cDNA is suspended in 0.2 ml of 0.1 M NaCl, 0.001 M EDTA, 0.01 M Tris buffer, pH 7.5 (NET buffer) and is applied onto a 4.4 ml of 5% to 20% w/v sucrose gradient (in NET buffer), on top of a 0.5 ml cushion of 30% sucrose. The DNA is centrifuged for 15 hours at 36,000 rpm at 4°C in a SW 50.1 rotor. Fractions (0.35 ml) are collected, and a sample of each fraction is counted. Fractions containing the center of the peak and heavier (greater than or equal to 600-800 base pairs) are pooled, tRNA is added to 10 µg/ml, and the nucleic acids precipitated with two volumes of ethanol.

Example 5    Construction of Hybrid Plasmids

Purified pBR322 DNA [Bolivar, F., Rodriguez, R.L., Greene, P.J., Betlach, M.C., Heyneker, H.L., Boyer, H.W., Crosa, J.H., and Falkow, S. (1977) Gene 2, 95-113. Sutcliffe, J.G. (1978) Nucleic Acids Res. 5, 2721-2728] is restricted with Pst I, phenol extracted, ethanol precipitated and dissolved in $H_2O$. The addition of dGTP to the pBR322 DNA (adjusted to 12.5 µg/ml) is performed in a reaction mixture containing 140 mM cacodylic acid, 30 mM Tris base, 110 mM KOH, 0.1 mM dTT, 0.1 mM dGTP, 1 mM $CoCl_2$, at pH 6.9, and 75 units/ml of terminal transferase for 5 minutes at 37°C. These conditions result in the addition of approximately 15 nucleotides to each end of the plasmid. The reaction is stopped by adding EDTA to 10 mM and ethanol precipitated in the presence of 0.1 M KCl [Gubbins, E.J., Maurer, R.A., Hartley, J.L., and Donelson, J.E. (1979) Nucleic Acids Res. 6, 915-930].

We chose to clone double-stranded cDNA synthesized from pituitary poly(A)-containing RNA since the majority of cDNA sequences synthesized from this RNA fraction could be expected to be complementary to PRL and GH mRNA.

The double-stranded cDNA is tailed with 12 µCi of $[\alpha-^{32}p]$-dCTP (429 Ci/mMole) using the same conditions described above except that the concentration of double-stranded cDNA is 2 µg/ml, dCTP is used instead of dGTP, and the terminal transferase concentration is 600 units/ml. The length of the oligo(dC) tails is approximately 15 nucleotides. The reaction is stopped and ethanol precipitated as described above, in the presence of 10 µg/ml yeast tRNA.

The oligo(dG)-tailed pBR322 DNA and the oligo(dC)-tailed double-stranded cDNA are diluted to 8 µg/ml and 0.6 µg/ml, respectively, in 100 mM NaCl, 10 mM Tris (pH 8.3), and 10 mM EDTA. The DNA's are annealed at a 1.8 fold molar excess of tailed pBR322, at a temperature gradient of 75°C to 37°C overnight and then at room temperature for 3 days [Jackson, D.A., Symons, R.H., and Berg, P. (1972) Proc. Natl. Acad. Sci. USA 69, 2904-2909].

The resulting hybrid plasmids are then used in the transformation process of Example 6.

Example 6      Transformation

The transformation procedure used is that described by Villa-Komaroff et al [Vila-Komaroff, L., Efstratiadis, A., Broome, S., Lomedico, P., Tizard, R., Naber, S.P., Chick, W.L., and Gilbert, W. (1978) Proc. Natl. Acad. Sci. USA 75, 3727-3731], except for the following modifications. The L-broth [Lennox, E.S. (1955) Virology 1, 190-206] is supplemented with 100 µg/ml diaminopimelic acid (DAP) and 20 µg/ml thymidine (THY). E. coli $_\chi$1776 cells are grown to a density

of $A_{600} = 0.3$. The transformation mixtures are composed of 0.2 ml cells and 0.016 ml recombinant plasmid (8.6 µg/ml). Three ml of brain heart infusion (BHI) soft agar (0.7%) is added to each aliquot of the transformation mixture before plating on BHI agar (1%) plates containing 15 µg/ml tetracycline (TET). When E. coli HB101 is used for transformation, the same procedure is applied, but the DAP and THY are omitted.

Example 7      Screening of Recombinant Plasmids

In order to identify clones containing GH sequences, it is necessary to prepare a hybridization probe specific for GH mRNA. This is accomplished by synthesizing cDNA (Example 2) to mRNA which is isolated after two sucrose gradient sedimentation steps as shown in Example 1. It is important to note that the mRNA prepared in this manner is highly enriched for GH mRNA sequences when assayed by cell-free translation in the wheat germ system.

The colony hybridization procedure is a modification of that originally described by Grunstein and Hogness [Grunstein, M. and Hogness, D.S. (1975) Proc. Natl. Acad. Sci. USA 72, 3961-3965]. Individual colonies are transferred to a millipore filter which is supported by 1.5% L-agar (containing DAP, THY and TET), and incubated overnight at 37°C. The bacteria are then lysed and their DNA fixed to the nitrocellulose filters as described by Gubbins et al., supra. The DNA-containing filters are pre-hybridized in a silanized glass petri dish for one hour at 37°C in 50% formamide containing 5X SSCP [Benton, W.D., and Davis, R.W. (1977) Science 196, 180-182], 0.5% SDS and 100 µg/ml sheared and denatured salmon sperm DNA. [$^{32}$p]-GH cDNA is then added ($10^6$ cpm/filter) and the hybridization is continued for at least 15 hours at 43°C. The filters are washed three times with 50% formamide containing 5X SSCP and 0.5% SDS and then two times with 2X SSCP as described by Benton and Davis, supra. Positive colonies are visualized by exposing the blotted filters overnight at -80°C to Kodak RP-X-OMAT film with DuPont Chronex lightning plus intensifying screen.

A highly positive colony is identified and the plasmid it contains is designated pLG23.

Example 8      Plasmid Amplification and Isolation

Each clone is rescreened by colony hybridization and then grown overnight in L-broth supplemented with DAP (100 µg/ml), THY (20 µg/ml) and TET (15 µg/ml). An aliquot of the overnight culture is then diluted 1:33 into one liter of L-broth + DAP + THY. When the cells have grown to a density corresponding to $A_{600}$ = 0.7-0.8, chloramphenicol (15 mg/ml in 100% ethanol) is added to a final concentration of 75 μg/ml, and then incubated for 15-20 hours. The cells are then pelleted, washed and frozen in dry ice-ethanol.

A slight modification of the procedures described by Godson and Sinsheimer [Godson, G.N., and Sinsheimer, R.L. (1967) Biochim. Biophys. Acta 149, 476-488] and Clewell and Helinski [Clewell, D.B., and Helinski, D.R. (1970) Biochemistry 9, 4428-4440] is used to lyse the cells with neutral detergent and to prepare cleared lysates. Frozen cell pellets from a one-liter culture are thawed and suspended in 10 ml of cold 25% sucrose, 0.05 M Tris (pH 8.0). Two ml of fresh, cold lysozyme (10 mg/ml) in 25% sucrose, 0.05 M Tris (pH 8.0) is added to the cells. The mixture is swirled intermittently on ice for 10 minutes. Then two ml of cold, 0.5 M EDTA is added, and the mixtures are again swirled intermittently on ice for five minutes. A total of 15 ml of NP-40 (well known surfactant) solution (0.2% NP-40, 6.25 mM EDTA, 50 mM Tris, pH 8.0) is added with mixing, and the tubes are left for 10 minutes at room temperature. The lysates are then centrifuged at 23,500 x g for 35 minutes at 4°C. The supernatants are treated with proteinase K (100 μg/ml) and SDS (0.1%) for 30 minutes at 37°C, diluted 1:1 with water, extracted with phenol-chloroform, and ethanol precipitated.

Recombinant plasmid DNA is further purified from RNA and E. coli chromosomal DNA by centrifugation in preparative CsCl bouyant density gradients essentially as described by Clewell and Helinski, supra. Aproximately 150 μg of plasmid DNA is routinely obtained from one liter of E. coli $_\chi$1776.

The recombinant plasmid containing the pLG23 sequence was subsequently transferred into the host E. coli HB101.

When recombinant plasmids are amplified in E. coli HB101, a modification of the alkaline extraction procedure described by Birnboim and Doly is used [Birnboim, H.C., and Doly, J. (1979) Nucleic Acids Res. 7, 1513-1523], giving a yield of over 1 mg plasmid per liter of culture.

The isolation, growth, and lysis of recombinant bacteria are all carried out in a P2 containment laboratory as specified by the NIH guidelines for recombinant DNA research.

-9- 3733

The following examples, concern procedures employed to obtain characterization data.

Example 9    Restriction Enzyme Analysis

The recombinant plasmids are restricted with Pst I, and the inserted DNA sequences are separated from the pBR322 plasmid on a 6% polyacrylamide gel. The inserts are eluted from the gel, dephosphorylated and 5' end labeled with $\gamma$-[$^{32}$p]-ATP as described by Maxam and Gilbert [Maxam, A., and Gilbert, W. (1977) Proc. Natl. Acad. Sci. USA 74, 560-564]. The end labeled inserts are restricted with different restriction enzymes, run on 6% polyacrylamide gels, and autoradiographed. Alternatively, the eluted Pst I fragments are restricted with restriction enzymes, run on 6% polyacrylamide gels, and visualized by staining with ethidium bromide.

The insert of clone pLG23 is approximately 841 bp long, and has two internal Pst I sites. Pst I digestion of the clone generates, besides the linear pBR322, 3 other fragments of 57 bp, 338 bp, and approximately 446 bp, as can be seen on 6% polyacrylamide gel. Each of the fragments is eluted from the gel, and is analyzed for restriction enzyme sites by 5' end labeling followed by autoradiography of the digestion products, or by staining the digestion products with ethidium bromide. In both cases, the digested fragments are separated on 6% polyacrylamide gels.

The preliminary 5' end labeling restriction analysis reveals that the 338 bp inserted fragment is restricted at least once with Alu I, Hae III, Hha I, Hpa II, and Hae II, and the 446 bp inserted fragment is restricted at least once with Alu I, Ava I, Hae III, Hha I, Sma I, Hpa II and Tha I. The inserted sequences did not have any EcoRI, Bam HI, Taq I, Pvu I, Hinc II, Kpn I, Sac I, Hind III, Sal I, Xho I, Bgl I, Bgl II, Xba I or Sst II restriction sites.

Further restriction analysis, done with ethidium bromide staining, reveals more information about the number of restriction sites and the size of the fragments in the inserted sequences. For example, the 57 bp inserted fragment is cut by Dde I once and gives approximately 19 and 38 bp fragments. The 338 bp inserted fragment is cut by Hha I into 67 bp, 100 bp, and 171 bp fragments. The 446 bp inserted fragment is cut once by Sma I to give a 125 bp and approximately 321 bp fragments, and twice by Hae III to give a 92 bp, approximately 159 bp, and 195 bp fragments.

Example 10    Selective Hybridization

The recombinant plasmids are linearized by EcoRI digestion, applied on nitrocellulose filters (SS-BA85) and hybridized to bovine pituitary poly A$^+$ RNA spiked with other mRNA's for 24 hours in the presence of 50% formamide. Following stringent washing, the hybridized RNA is eluted at 65°C in the presence of 90% formamide and translated in a wheat germ cell free translation system.

Plasmid pLG23 is further characterized as a GH clone by selective hybridization. The recombinant plasmid is linearized, bound to nitrocellulose filter, and hybridized to a mixture of bovine pituitary poly A$^+$ RNA, globin mRNA, GH mRNA, and ovalbumin mRNA. Following stringent washing, the hybridized RNA is eluted and translated in a wheat germ cell free translation system. The major translation product is pregrowth hormone.

Example 11    DNA Sequence Analysis

The chemical modification and cleavage reactions for DNA described by Maxam and Gilbert, supra, are used to sequence restriction fragments of the cloned DNA. Thin sequencing gels described by Sanger and Coulson [Sanger, F.A., and Coulson, A.R. (1978) FEBS Letters 87, 107-110] are employed for all analyses.

Partial Nucleotide Sequence of the pLG23 Insert.    The DNA isolated from the recombinant plasmid pLG23 is digested with Pst I and then electrophoresed in a 6% polyacrylamide gel [Peacock, A.C., and Dingman, C.W. (1968) Biochemistry 7, 668-674]. The insert is eluted from the gel, dephosphorylated with bacterial alkaline phosphatase and end-labeled with polynucleotide kinase [Maxam and Gilbert, supra]. The labeled 338 bp insert fragment is digested with Hha I. The products of this digestion are separated on a 6% polyacrylamide gel. The 67 bp and 171 bp fragments are sequenced according to Maxam and Gilbert, supra. Following is a summary of the nucleotide sequence that is obtained from the above fragments and the amino acids predicted from one potential reading frame of this sequence. The correlation between the known amino acid sequence of bovine pre GH [Hunt, et al., supra, and Lingappa, et al., supra], and the amino acid sequence deduced from the inserted sequence of pLG23, permits a positive identification of this clone as one containing the sequence for pre GH.

```
          Leu Leu Leu Ala Phe Ala Leu Leu Cys Leu Pro Trp Thr Gln
5'...C CUG CUC CUG GCU UUC GCC CUG CUC UGC CUG CCC UGG ACU CAG

     Val Val
     GUG GUG G...

        70                                        80
   Gln Gln Lys Ser Asp Leu Glu Leu Leu Arg Ile Ser Leu Leu Leu Ile Gln
...CAG CAG AAA UCA GAC UUG GAG CUG CUU CGC AUC UCA CUG CUC CUC AUC CAG

     Ser Trp Leu ...3'
     UCG UGG CU
```

The partial sequencing of the growth hormone clone and the restriction analysis data locates the two Pst I recognition sites at a.a. -24 to -22 (signal peptide) and a.a. 90-91 of the growth hormone [Hunt et al., supra, and Lingappa, et al., supra]. Thus, the 338 bp Pst I fragment is located in the center of the cloned sequence, the 57 bp Pst I fragment is located at the 5' end of the sequence, and the 446 bp Pst I fragment is located at the 3' end of the growth hormone insert.

Example 12      Orientation of the GH Insert in Plasmid pBR322

In order to find in which orientation the GH insert is located within the β lactamase gene of the plasmid pBR322, a double digestion analysis is performed. The recombinant plasmid of clone pLG23 is digested with both Pvu I and Sma I restriction enzymes. Pvu I cuts once in the pBR322 plasmid at position 3734, and doesn't cut within the GH insert. Sma I cuts once in the GH insert but doesn't restrict the pBR322 DNA. The Sma I recognition site (5' CCCGGG) is in the 446 bp Pst I fragment of the insert, at position 132-134 of the GH a.a. sequence. Sma I digestion of the 446 bp fragment generates 125 bp and approximately 321 bp fragments. The GH sequence is inserted in the Pst I site (position 3608) of plasmid pBR322 which is the codon for a.a. 182 of the β lactamase gene [Villa-Komaroff et al., supra, and Erwin, C.R., Maurer, R.A., and Donelson, J.E. (1980) Nucleic Acids Res. 8, 2537-2546]. Thus, if the GH insert is oriented in a 5' to 3' clockwise direction in respect to the coding sequence, one expects to get a 447 bp fragment and a 4756 bp fragment following Pvu I-Sma I double digestion. However, if the GH sequence is inserted in a 5' to 3' counterclockwise orientation, as does the β-lactamase gene [Villa-Komaroff et al., supra, and Erwin et al., supra], one expects 646 and 4557 bp fragments resulting from the above double restriction.

When pLG23 DNA is restricted with Pvu I and Sma I, a high molecular weight band and a lower molecular weight band are observed in a 6% polyacrylamide gel, and on a 1% agarose gel. The lower MW band corresponds to about 646 bp in length. Thus, the inserted sequence is in a 5' to 3' counterclockwise orientation in the Pst I site of pBR322, and is probably transcribed and translated with part of the β lactamase gene as a fusion protein. Whether the GH insert is indeed expressed as a GH - β lactamase fusion protein depends on the reading frame of this insert within the β lactamase gene.

The amino acid sequence of bGH from residue 1 to 191 is as follows:

```
 1                              10
Ala Phe Pro Ala Met Ser Leu Ser Gly Leu Phe Ala Asn Ala Val Leu Arg

        20                              30
Ala Gln His Leu His Gln Leu Ala Ala Asp Thr Phe Lys Glu Phe Glu Arg

                    40                              50
Thr Tyr Ile Pro Glu Gly Gln Arg Tyr Ser Ile Gln Asn Thr Gln Val Ala

                            60
Phe Cys Phe Ser Glu Thr Ile Pro Ala Pro Thr Gly Lys Asn Glu Ala Gln

    70                              80
Gln Lys Ser Asp Leu Glu Leu Leu Arg Ile Ser Leu Leu Leu Ile Gln Ser

            90                              100
Trp Leu Gly Pro Leu Gln Phe Leu Ser Arg Val Phe Thr Asn Ser Leu Val

                        110
Phe Gly Thr Ser Asp Arg Val Tyr Glu Lys Leu Lys Asp Leu Glu Glu Gly

120                             130
Ile Leu Ala Leu Met Arg Glu Leu Glu Asp Gly Thr Pro Arg Ala Gly Gln

        140                             150
Ile Leu Lys Gln Thr Tyr Asp Lys Phe Asp Thr Asn Met Arg Ser Asp Asp

                        160                             170
Ala Leu Leu Lys Asn Tyr Gly Leu Leu Ser Cys Phe Arg Lys Asp Leu His

                    180
Lys Thr Glu Thr Tyr Leu Arg Val Met Lys Cys Arg Arg Phe Gly Glu Ala

        190 191
Ser Cys Ala Phe
```

Pre-bGH has the following partial amino acid sequence added to the amino terminus of the bGH sequence (Lingappa et al., supra):

13

Met Met ... ... ... Pro ... ... ... Leu Leu Leu ... Phe ... Leu
Leu ... Leu Pro ... ... ... ... ... ...

Plasmid pLG23 in an E. coli host, and E. coli HB101, have been deposited in the permanent collection of the Northern Regional Research Laboratory (NRRL), U.S. Department of Agriculture, Peoria, Illinois, U.S.A.

This deposit of HB101 (pLG23) has the accession number NRRL B-12436, and a deposit of E. coli HB101, a known and widely-available host microbe, has the accession number NRRL B-11371. Both deposits were made on 3rd May 1981.

There are other well known hosts which can be used instead of E. coli HB101. For example, B. subtilis, Streptomyces species and yeast can be used.

Other vectors (or vehicles) which can be used in the invention are pBR313 which codes for ampicillin and tetracycline resistance, pSC101 which codes for tetracycline resistance, pCR11 which codes for kanamycin resistance, λ bacteriophage vectors, for example, charon phages, and yeast 2μ plasmid DNA.

The utility of plasmid pLG23 is readily apparent to a person skilled in the art. It can be used to direct the synthesis of bovine growth hormone in a micro-organism host, e.g., E. coli; see Keshet et al., Nucleic Acids Res. 9, supra.

14

CLAIMS

1.    Plasmid pLG23.

2.    E. coli HB101 (pLG23), having the deposit accession number NRRL B-12436.

3.    A micro-organism modified to contain a nucleotide sequence coding for the amino-acid sequence of pre-bGH.

4.    A micro-organism modified to contain a nucleotide sequence coding for the amino-acid sequence of bovine growth hormone.

5.    A DNA transfer vector comprising a nucleotide sequence coding for the amino-acid sequence of pre-bGH.

6.    A DNA transfer vector comprising a nucleotide sequence coding for bovine growth hormone.

7.    A transfer vector according to claim 5 or claim 6, which has been replicated in a micro-organism.

8.    A transfer vector according to claim 7, which is a plasmid, and wherein the micro-organism is a bacterium.

9.    A micro-organism or transfer vector according to any of claims 3 to 8, wherein the micro-organism is an E. coli.

10.    A transfer vector according to claim 8, wherein the bacterium is Escherichia coli χ 1776 or Escherichia coli HB101, and the plasmid is pBR322.

11.    A process for preparing bGH, which comprises culturing a bacterium according to claim 4 or claim 9.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | THE JOURNAL OF BIOLOGICAL CHEMISTRY, vol. 255, no. 16, 25th August 1980, pages 7521-7524, USA W.L. MILLER et al.: "Molecular cloning of DNA complementary to bovine growth hormone mRNA" * Whole article * | 1-11 | C 12 N 15/00 C 12 P 21/02 C 12 N 1/20 // C 12 R 1/19 |
| D,X | NUCLEIC ACIDS RESEARCH, vol. 9, no. 1, 1981, pages 19-30, IRL Press Ltd., G.B. E. KESHET et al.: "Cloning of bovine growth hormone gene and its expression in bacteria" * Whole article * | 1-11 | |
| P,X | EP-A-0 047 600 (THE REGENTS OF THE UNIVERSITY OF CALIFORNIA) * Abstract; page 1, line 1 - page 6, line 20; page 10, line 6 - page 19, line 3; example 1 * | 1-11 | TECHNICAL FIELDS SEARCHED (Int. Cl. ³) |
| X | EP-A-0 001 929 (GENENTECH, INC.) * Abstract; page 6, lines 20-31; page 8, line 25 - page 12, line 33; claim 6 * | 1-11 | C 12 N C 12 P C 12 R |
| X | EP-A-0 012 494 (THE REGENTS OF THE UNIVERSITY OF CALIFORNIA) * Abstract; page 11, line 11 - page 13, line 11; page 25, lines 4-7; claims 1,6,16,19,21,25 * | 1-11 | |

-/-

The present search report has been drawn up for all claims

| Place of search | Date of completion of the search | Examiner |
|---|---|---|
| THE HAGUE | 01-09-1982 | DE LUCA G.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

**European Patent Office**

**EUROPEAN SEARCH REPORT**

Application number

EP 82 30 2804

| | DOCUMENTS CONSIDERED TO BE RELEVANT | | | Page 2 |
|---|---|---|---|---|

| Category | Citation of document with indication, where appropriate, of relevant passages | Relevant to claim | CLASSIFICATION OF THE APPLICATION (Int. Cl. ³) |
|---|---|---|---|
| P,X | GB-A-2 073 245 (YEDA RESEARCH)<br>* Whole document * | 1-11 | |
| | ----- | | |
| | | | **TECHNICAL FIELDS SEARCHED (Int. Cl. ³)** |

The present search report has been drawn up for all claims

| Place of search | Date of completion of the search | Examiner |
|---|---|---|
| THE HAGUE | 01-09-1982 | DE LUCA G.M. |

EPO Form 1503. 03.82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document